# EUROPÄISCHE PATENTSCHRIFT

(11) **EP 2 999 463 B1**
(45) Veröffentlichungstag und Bekanntmachung des Hinweises auf die Patenterteilung: **18.04.2018**
(21) Anmeldenummer: 14729853.3
(22) Anmeldetag: 16.05.2014
(51) Int. Cl.: A61K 9/70, A61F 13/00, A61F 13/02

(54) **PFLASTER MIT GEKERBTER ABZIEHFOLIE**
PLASTER COMPRISING PRE-GROOVED PEELING FILM
PATCH À FILM PELABLE ENTAILLÉ

(30) Priorität: 23.05.2013 DE 102013008727
(43) Veröffentlichungstag der Anmeldung: 30.03.2016
(73) Patentinhaber: AMW GmbH, 83627 Warngau (DE)
(72) Erfinder: KAFFL, Hubert, 83627 Warngau (DE)
(74) Vertreter: Beckord & Niedlich Patentanwälte PartG mbB
(86) Internationale Anmeldenummer: PCT/EP2014/001331
(87) Internationale Veröffentlichungsnummer: WO 2014/187548

(56) Entgegenhaltungen:
- EP-A1- 2 042 138
- EP-A1- 2 377 498
- EP-A1- 2 489 339
- US-A- 5 820 877

## Beschreibung

Transdermale therapeutische Systeme weisen regelmäßig eine Folie auf, die die selbstklebende und gegebenenfalls wirkstofflialtige Schicht schützt und vor der Applikation zu entfernen ist und als Abziehfolie bezeichnet wird. Es sind viele Vorschläge gemacht worden, das Abziehen der Folie zu erleichtern.

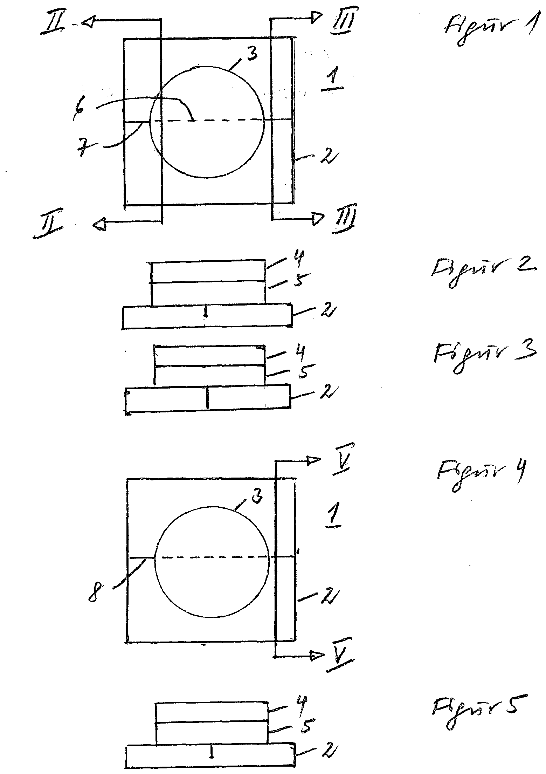

So ist die Abziehfolie gemäß EP 2 481 443 A1 ([0081] mit Figur 5A) mit einer Zeile von Einschnitten und gemäß Patentanmeldung JP HEI 8-112 305 (vgl. EP 1 552 821 B1 [0004]) oder EP 0 943 138 B1 ([0014]) mit einer Zeile von Perforationen versehen, so dass die Folie an der Zeile reißen und auseinander gezogen werden kann, um die selbstklebende Schicht freizugeben.

Auch hat man zwei Folien bzw. zwei Folienhälften vorgesehen, die gemeinsam die wirkstofflialtige Schicht abdecken, jede Folie bzw. Folienhälfte für sich jedoch nur einen Teilbereich der selbstklebenden Schicht, beispielsweise etwa zur Hälfte, indem die abdeckende Folie mit einem halbierenden Schnitt ("half cut") gehälftelt ist; vgl. EP 0 943 138 B1 ([0014]), EP 1258 517 B1 ([0029] mit Figur 1), EP 1 411 906 B1 (Figur 2), EP 2 206 759 B1 (Figur 1), EP 2 481 443 A1 ([0082] mit Figur 5B). Sofern sich die beiden Folien überlappen, wird einem Austritt der Matrix der gegebenenfalls wirkstoffhaltigen Schicht, in der Regel eines selbsthaftenden Klebers, vorgebeugt; vgl. EP 1 180 023 B1 (Figur 1), EP 1 967 170 A1 (Figur 1.2), EP 2 340 815 B1 (Figur 4).

Immer wieder weist der Stand der Technik darauf hin, dass Matrix bzw. selbsthaftender Kleber durch die Abziehfolie austreten kann, sofern die Abziehfolie nicht geschlossen ist; vgl. EP 1 180 023 B1 (Spalte 2 Zeilen 46-50), EP 1258 517 B1 ([0034], [0036], [0043]), EP 1 552 821 B1 ([0004], [0005], [0006]), EP 2 042 138 A1 ([0032]).

So hat man weiterhin vorgeschlagen, die Abziehfolie nicht mit einem Schnitt oder in Form von zwei Folienhälften vorzusehen, sondern die Abziehfolie nur mit einer Einkerbung zu versehen; vgl. EP 0 943 138 B1 ([0014]), EP 1258 517 B1 ([0036] mit Figur 2 und [0042] mit Figur 4), EP 1 552 821 B1 ([0015] mit Figuren 1-2), EP 1 915 976 A1 ([0010]), EP 2 042 138 A1 ([0031] mit Figur 1), EP 2 078 517 A2 ([0016] mit Figur 2) und EP 2 489 340 A1 ([0067] mit Figur 5).

EP 0 943 138 B1 beschreibt ein Pflaster mit Abdeckfolie, Klebstoffschicht und Abziehfolie, wobei die Abziehfolie Einkerbungen als Sollbruchlinien aufweisen kann ([0014]), wobei die Sollbruchlinien von unten bzw. von der Seite der Abziehfolie her eingestanzt werden, die der Klebstoffschicht abgewandt ist (Seite 2 links Zeilen 31-34).

Nach EP 1 258 517 B1 wird ein Klebelement aus einem Träger bzw. einer Deckfolie (1), Klebschicht (2) und Abziehfolie (3) mit einem Einschnitt ("half cut") vorgesehen, der als hälftelnder Schnitt oder Einkerbung ausgebildet sein kann (Ansprüche 9, 11 und 13). Der hälftelnde Schnitt bzw. die Einkerbung kann grundsätzlich bis zu einer Tiefe vorgesehen werden, die mehr als 14/15 der Dicke der Abziehfolie beträgt, jedoch weniger als deren Dicke plus der Dicke der Klebschicht (Anspruch 2). Die Einkerbung wird also von der der Klebeschicht abgewandten Seite der Abziehfolie her vorgenommen, so dass die Einkerbung von da her bis in die Klebeschicht eindringen kann. Die Einkerbung muss aber nicht bis in die Klebeschicht geführt werden, sie kann auch mit einer Tiefe von weniger als der Dicke der Abziehfolie, jedoch mehr als 14/15 ihrer Dicke vorgesehen werden.

EP 1 552 821 B1 ([0015] und [0017] mit Figuren 1-2) beschreibt eine Abziehfolie für ein Pflaster aus dieser Folie, einer Grundmittelschicht und einem Träger. Dabei ist die Abziehfolie mit einem Kantenabschnitt eines Presselements in einem Abschnitt, dem sogenannten Abreißlinien-Abschnitt, derart verpresst, dass die Stärke des verpressten Abschnitts geringer ist als die Stärke der Abziehfolie (Ansprüche 1 und 5). So kann eine PE-Abziehfolie eines Pflasters aus der Folie, einem Salbenauftrag und einem abdeckenden Gewebe mit einem heißen Heißsiegel-Draht in Berührung gebracht und der sogenannte Abreißlinien-Abschnitt vorgesehen werden (Beispiel 5).

EP 1 915 976 A1 ([0010]) beschreibt ein Pflaster mit einer Abziehfolie, die eine Trennzone aufweist, die einen Bereich mit einem dünnen Schnitt bzw. dünner Schnitte ("thin cut part") aufweisen kann, die nicht durch die Abziehfolie hindurch geführt sind, bei denen es sich um eine Einkerbung ("half cut") handeln kann ([0010]), wobei benachbart zur Trennzone zusätzlich ein oder mehrere vorgeschnittene Bereiche ("precut part") vorgesehen sein können, bei denen es sich gleichfalls um Einkerbungen handeln kann [0013]. Stand der Technik, nach dem die Abziehfolie eines Pflasters mit Einkerbungen versehen werden kann, wird vorausgesetzt.

EP 2 042 138 A1 beansprucht ein Pflaster mit Deckfolie, Klebschicht und Abziehfolie, bei dem die Abziehfolie mit einer der Klebschicht abgewandten Einkerbung einer Tiefe versehen ist, die mehr als die halbe Dicke, aber weniger als die Dicke der Folie misst (Anspruch 1).

EP 2 078 517 A2 beansprucht ein Verfahren zur Verwendung eines derartigen Pflasters (Ansprüche 1 bis 5) sowie ein in dem Verfahren zu verwendendes Pflaster (Anspruch 6).

EP 2 489 340 A1 beansprucht ein Klebepflaster mit einer Trägerfolie, einer Klebemittelschicht und einer Abziehfolie (16 in Figur 5), wobei die Abziehfolie eine Schwachzone (20 in Figur 5) in Form einer Kerbe ("half-cut" gemäß [0067]) aufweist, und ferner eine Folie (18 in Figur 5) mit einer Schwachzone (gleichfalls 20 in Figur 5) trägt, die entsprechend der Schwachzone der Abziehfolie positioniert ist und mit einem Teilbereich auf der Abziehfolie fixiert ist und mit einem anderen Teilbereich als Griff (bzw. pinching piece) dient. Die Schwachzonen (20 in Figur 5) können dadurch vorgesehen werden, dass die Abziehfolie mit der von ihr getragenen weiteren Folie einem Laser ausgesetzt wird ([0067]).

Der Erfindung liegt nun die Aufgabe zugrunde, den Stand der Technik, bei dem die einteilige Abziehfolie mit einem "half-cut" bzw. einer Nut oder Kerbe versehen wird, zu verbessern.

Erfindungsgemäß wird dazu ein Pflaster, insbesondere transdermales vorzugsweise therapeutisches System mit einer
- Deckfolie,
- einer Schicht (Matrix-Schicht), bestehend aus oder umfassend eine wirkstofflialtige selbstklebende Matrix,
sowie
- einer einteiligen Abziehfolie vorgesehen, wobei
die Abziehfolie mit einer Nut versehen ist, die auf ihrer der Matrix-Schicht zugewandten Seite vorgesehen ist, und wobei entweder die Nut den Rand bzw. die Peripherie der Matrix-Schicht und einer fakultativen Klebemittel-Schicht erreicht oder sich darüber hinaus erstreckt oder die Peripherie der Abziehfolie erreicht.

Bei dem erfindungsgemäßen Pflaster, insbesondere transdermalen vorzugsweise therapeutischen System beträgt die Tiefe der Nut mindestens 1/2, vorzugsweise 2/3 und insbesondere 14/15 der Dicke der Abziehfolie, jedoch stets weniger als deren Dicke.

Ferner kann bei dem erfindungsgemäßen Pflaster, insbesondere transdermalen vorzugsweise therapeutischen System die Nut geradlinig ausgebildet sein und vorzugsweise etwa die Mitte der Abziehfolie durchqueren.

Ferner kann bei dem erfindungsgemäßen Pflaster, insbesondere transdermalen vorzugsweise therapeutischen System die Nut die Peripherie der Matrix-Schicht und der fakultativen oder der obligatorischen Klebemittel-Schicht erreichen oder sich darüber hinaus erstrecken, jedoch nicht die Peripherie der Abziehfolie erreichen, wobei die Abziehfolie, beginnend an den beiden entgegengesetzten Enden der Nut bis an die Peripherie der Abziehfolie voll durchgetrennt ist.

Ferner kann bei dem erfindungsgemäßen Pflaster, insbesondere transdermalen vorzugsweise therapeutischen System die Abziehfolie aus Polyethylen (PE), Polypropylen (PP) oder Polyester bestehen, insbesondere Polyethylenterephthalat.

Ferner kann bei dem erfindungsgemäßen Pflaster, insbesondere transdermalen vorzugsweise therapeutischen System die Abziehfolie eine Dicke von 25 bis 200 und insbesondere 50 bis 150 Mikrometer aufweisen.

Schließlich kann bei dem erfindungsgemäßen Pflaster, insbesondere transdermalen vorzugsweise therapeutischen System die Abziehfolie die Deckfolie mit Matrix-Schicht und fakultativer Klebemittel-Schicht allseitig überragen.

Das erfindungsgemäße Pflaster, insbesondere transdermale System kann sowohl für schützende, kosmetische oder therapeutische Zwecke vorgesehen sein. Es kann also einen oder mehrere Wirkstoffe enthalten.

Wenn bei der Pflasterherstellung vor der Vereinzelung der einzelnen Pflaster für deren Abziehfolien ein Band verwendet wird, aus dem die einzelnen Abziehfolien isoliert werden, so wird das Band vorzugsweise in Längsrichtung mit aufeinanderfolgenden Nuten einer Länge versehen,
- die der Länge oder Breite einer vereinzelten Abziehfolie entsprechen oder
- die dem Durchmesser einer vereinzelten Deckfolie entsprechen, wobei sich bei dieser Ausführungsform an die Nuten beidseitig Schnitte anschließen, die bis an den Rand der vereinzelten Abziehfolie geführt werden bzw. geführt sind und sie durchtrennen. Für die Stabilität des Bandes ist es förderlich, jede Nut mit den fakultativen Schnitten in Längsrichtung des Bandes vorzusehen.

Indem erfindungsgemäß die Abziehfolie auf der Seite mit einer Nut oder Kerbe bzw. Einkerbung versehen ist, die der Matrix-Schicht zugewandt ist, ergibt sich der Vorteil, dass die der Matrix-Schicht abgewandte Seite der Abziehfolie eben und glatt ist, so dass sich eventuelle Verletzungen leicht erkennen lassen. Auch können alle Kerb- und SchneidWerkzeuge bei der Herstellung der vereinzelten Pflaster von der Seite der Deckfolien-Bahn bzw. der Pflaster-Deckfolie her eingesetzt werden.

**Figur 1** ist eine Draufsicht auf ein erfindungsgemäßes Pflaster 1 mit Abziehfolie 2 und Bauelement 3 (umfassend Abdeckfolie 4 und Matrix-Schicht 5). **Figur 2** ist eine Ansicht des Schnitts II-II in **Figur 1** und zeigt, dass die Nut 6 bis an den Rand des Bauelements 3 geführt ist. **Figur 3** ist eine Ansicht des Schnitts III-III in **Figur** 1 und zeigt, dass die Nut 6 von den Rändern des Bauelements 3 beidseitig bis an den Rand der Abziehfolie 2 jeweils als Schnitt 7 fortgesetzt ist.

**Figur 4** ist wiederum eine Draufsicht auf ein erfindungsgemäßes Pflaster 1 mit Abziehfolie 2 und Bauelement 3 (umfassend Abdeckfolie 4 und Matrix-Schicht 5). **Figur 5** ist eine Ansicht des Schnitts V-V in **Figur 4** und zeigt, dass die Nut 8 über die Abziehfolie 2 bis an deren Ränder geführt ist.

Bei beiden Ausführungsformen der **Figuren 1-3** und **4-5** sind die Nute 6 und 8 dem Bauelement 3 zugewandt.

Nachstehend wird die Erfindung durch ein Beispiel näher erläutert.

### Beispiel 1

Es wird eine einseitig silikonisierte Polyesterfolie (Polyethylenterephthalat; z.B. Primeliner PET 75 mikro m 1S) in Form eines Bandes mit Einkerbungen bzw. Nuten quer zur Längsrichtung des Bandes versehen. Diese Nuten werden in derartigen Abständen vorgesehen, dass sie die der Folie später schichtförmig aufliegende Matrix (Matrixschicht) mit ihrer Deckfolie in Draufsicht auf die herzustellenden Pflaster mitteln. Die Nuten sind derart ausgebildet, dass sie der Matrixschicht plus Deckfolie der herzustellenden Pflaster zugewandt sind und am Rand der Matrixschicht plus Deckfolie der herzustellenden Pflaster enden.

Außerdem werden 0,45 g (9 %) Rotigotin (freie Base) in 1 g Aceton vorgelöst. Die Vorlösung wird zu einer Mischung aus 0,2 g (4 %) Phosphatpuffer (pH 6), 0,2 g (4 %) Silikonöl (Medical Fluid), 0,2 g (4 %) Sorbitan-monostearat (Span 60) und 1 g Aceton gegeben. Die resultierende Mischung wird zu einer Lösung eines Haftklebers gegeben (z.B. 3,95 g (79 %) DuroTak 6911A), wonach 12 h gerührt wird. Die erhaltene Beschichtungsmasse wird auf die bandförmige silikonisierte Polyesterfolie aufgetragen. Danach wird bei 70 °C 20 min getrocknet und ein bandförmiges 2-schichtiges Laminat erhalten.

Auf das bandförmige 2-schichtige Laminat wird danach eine bandförmige Polyesterfolie (z.B. Hostaphan MN 23 DMF) aufkaschiert. Danach werden durch die Deckfolie und die Matrixschicht des nun 3-schichtigen Laminats kreisförmige Schnitte gestanzt, die die Matrix-Schicht und Deckfolie herzustellender Pflaster umreißen. Danach wird das anfallende bandförmige Gitter aus Deckfolie und Matrixschicht abgezogen. Schließlich werden rechteckige Schnitte durch die Abziehfolie vorgesehen, die fertige Pflaster definieren und im Abstand um die zuvor kreisförmig vorgesehenen Schnitte verlaufen. Das dabei anfallende Gitter der Abziehfolie wird von den fertigen Pflastern separiert.

### Beispiel 2

Es wird Beispiel 1 mit der Abänderung wiederholt, dass die bandförmige silikonisierte Polyesterfolie fluchtend mit den Nuten auf beiden Seiten jeder Nut bis zum Rand des Bandes durchgeschnitten wird.

## Patentansprüche

1. Pflaster mit einer
- Deckfolie,
- einer Schicht (Matrix-Schicht), bestehend aus oder umfassend eine wirkstoffhaltige selbstklebende Matrix, sowie
- einer einteiligen Abziehfolie, wobei
die Abziehfolie mit einer Nut versehen ist, die auf ihrer der Matrix-Schicht zugewandten Seite vorgesehen ist, und wobei entweder
die Nut die Peripherie der Matrix-Schicht erreicht oder sich darüber hinaus erstreckt oder
die Nut die Peripherie der Abziehfolie erreicht
und wobei
die Tiefe der Nut mindestens 1/2, vorzugsweise 2/3 und insbesondere 14/15 der Dicke der Abziehfolie, jedoch stets weniger als deren Dicke beträgt.

2. Pflaster gemäß Anspruch 1, ferner umfassend eine selbstklebende Klebemittel-Schicht.

3. Pflaster, insbesondere transdermales vorzugsweise therapeutisches System, nach einem der Ansprüche 1 und/oder 2, bei dem die Nut geradlinig ausgebildet ist und vorzugsweise etwa die Mitte der Abziehfolie durchquert.

4. Pflaster, insbesondere transdermales vorzugsweise therapeutisches System, nach Anspruch 1, 2 und/oder 3, bei dem die Nut die Peripherie der Matrix-Schicht erreicht oder sich darüber hinaus erstreckt, jedoch nicht die Peripherie der Abziehfolie erreicht, wobei die Abziehfolie, beginnend an den beiden entgegengesetzten Enden der Nut bis an die Peripherie der Abziehfolie, voll durchgetrennt ist.

5. Pflaster, insbesondere transdermales vorzugsweise therapeutisches System, nach mindestens einem der Ansprüche 1, 2, 3 und/oder 4, wobei die Abziehfolie aus Polyethylen (PE), Polypropylen (PP) oder Polyester besteht, insbesondere Polyethylenterephthalat.

6. Pflaster, insbesondere transdermales vorzugsweise therapeutisches System, nach mindestens einem der Ansprüche 1, 2, 3, 4 und/oder 5 mit einer Abziehfolie einer Dicke von 25 bis 200 und insbesondere 50 bis 150 Mikrometer.

7. Pflaster, insbesondere transdermales vorzugsweise therapeutisches System, nach mindestens einem der vorhergehenden Ansprüche, bei dem die Abziehfolie die Deckfolie mit Matrix-Schicht allseitig überragt.

## Claims

1. A plaster having
- a cover film,
- a layer (matrix layer) consisting of or comprising an active-substance-containing, self-adhesive matrix, and
- a single-part release liner wherein
the release liner is provided with a groove, which is provided on the side of the release liner which faces the matrix layer, and wherein either
the groove reaches the periphery of the matrix layer or extends beyond same or
the groove reaches the periphery of the release liner
and wherein
the depth of the groove is at least 1/2, preferably 2/3 and in particular 14/15 of the thickness of the release liner, but always less than the thickness of the release liner.

2. The plaster according to claim 1, further comprising a self-adhesive adhesive agent layer.

3. The plaster, in particular transdermal, preferably therapeutic system, according to any one of claims 1 and/or 2, in which the groove is straight and preferably crosses approximately the centre of the release liner.

4. The plaster, in particular transdermal, preferably therapeutic system, according to any one of claims 1, 2 and/or 3, in which the groove reaches the periphery of the matrix layer or extends beyond same but does not reach the periphery of the release liner, wherein the release liner, from the two opposing ends of the groove to the periphery of the release liner, is completely cut through.

5. The plaster, in particular transdermal, preferably therapeutic system, according to any one of claims 1, 2, 3 and/or 4, wherein the release liner consists of polyethylene (PE), polypropylene (PP) or polyester, in particular polyethylene terephthalate.

6. The plaster, in particular transdermal, preferably therapeutic system, according to any one of claims 1, 2, 3, 4 and/or 5, having a release liner with a thickness of 25 to 200 micrometres, in particular 50 to 150 micrometres.

7. The plaster, in particular transdermal, preferably therapeutic system, according to any one of the preceding claims, in which the release liner projects beyond the top film with the matrix layer on all sides.

## Revendications

1. Sparadrap, comprenant
- un film de couverture,
- une couche (couche matricielle), constituée de ou comprenant une matrice autocollante contenant un principe actif, ainsi
- qu'un film détachable, en un élément,
le film détachable étant doté d'une rainure, qui est prévue sur son côté faisant face à la couche matricielle et soit
la rainure atteignant la périphérie de la couche matricielle ou s'étendant au-delà de cette dernière, ou
la rainure atteignant la périphérie du film détachable
et
la profondeur de la rainure correspondant au moins à 1/2, de préférence aux 2/3 et notamment à 14/15 de l'épaisseur du film détachable, tout en était toujours inférieure à l'épaisseur de celui-ci.

2. Sparadrap selon la revendication 1, comprenant par ailleurs une couche autocollante d'agent adhésif.

3. Sparadrap, notamment système transdermique, de préférence thérapeutique selon l'une quelconque des revendications 1 et/ou 2, sur lequel la rainure est conçue de forme rectiligne et traverse de préférence approximativement le milieu du film détachable.

4. Sparadrap, notamment système transdermique, de préférence thérapeutique selon la revendication 1, 2 et/ou 3, sur lequel la rainure atteint périphérie de la couche matricielle ou s'étend au-delà de celle-ci, mais n'atteint pas la périphérie du film détachable, à partir des deux extrémités opposées de la rainure le film détachable, étant totalement sectionnée jusqu'à la périphérie du film détachable.

5. Sparadrap, notamment système transdermique, de préférence thérapeutique selon au moins l'une quelconque des revendications 1, 2, 3 et/ou 4, le film détachable étant en polyéthylène (PE), en polypropylène (PP) ou en polyester, notamment en polytéréphtalate d'éthylène.

6. Sparadrap, notamment système transdermique, de préférence thérapeutique selon au moins l'une quelconque des revendications 1, 2, 3, 4 et/ou 5, comprenant un film détachable d'une épaisseur de 25 à 200 et notamment de 50 à 150 micromètres.

7. Sparadrap, notamment système transdermique, de préférence thérapeutique selon l'une quelconque des revendications précédentes, sur lequel par la couche matricielle, le film détachable saillit de toute part par-dessus le film de couverture.
